# EUROPEAN PATENT SPECIFICATION

(11) **EP 2 294 971 B1**
(45) Date of publication and mention of the grant of the patent: **05.10.2016**
(21) Application number: 10181936.5
(22) Date of filing: 19.08.2005
(51) Int. Cl.: A61B 5/145, G01J 3/02, G01N 33/487, G01N 21/84

(54) **AN INSTRUMENT WITH A MEMORY CARD UPDATING MEASURMENT ALGORITHMS AND METHODS OF USING THE SAME**
INSTRUMENT MIT SPEICHERKARTEN-AKTUALISIERUNGSMESSLALGORITHMEN UND ANWENDUNGSVERFAHREN DAFÜR
INSTRUMENT AVEC UNE CARTE MEMOIRE METTANT A JOUR DES ALGORITHMES DE MESURE ET PROCEDES D'UTILISATION DE CELUI-CI

(30) Priority: 24.08.2004 US 603951 P
(43) Date of publication of application: 16.03.2011
(62) Divisional of application: 05789185.5
(73) Proprietor: Ascensia Diabetes Care Holdings AG, 4052 Basel (CH)
(72) Inventor: Dodson, Neil A., South Bend, IN 46635 (US)
(74) Representative: Cohausz & Florack

(56) References cited:
- EP-A2- 0 640 978
- WO-A1-00/27276
- US-A- 5 077 476

## Description

### FIELD OF THE INVENTION

The present invention generally relates to a programming strip to be used in an instrument and, more particularly, to a programming strip that is adapted to provide updated information to a processor of the instrument that determines the concentration of an analyte (*e.g.,* glucose).

### BACKGROUND OF THE INVENTION

The quantitative determination of analytes in body fluids is of great importance in the diagnoses and maintenance of certain physiological abnormalities. For example, lactate, cholesterol and bilirubin should be monitored in certain individuals. In particular, determining glucose in body fluids is important to diabetic individuals who must frequently check the glucose level in their body fluids to regulate the glucose intake in their diets. To determine what, if any, medication (*e.g.,* insulin) needs to be administered, a reagent-test sensor may be used for testing a fluid such as a sample of blood. The test sensors typically have been used in an instrument, including sensor-dispensing instruments. The instruments typically include a processor that is initially programmed at a manufacturing facility.

To properly read and determine the information obtained from the reagent-test sensor, new information may periodically be needed to update the processor of the instrument. This information may be in the form of reprogramming the existing algorithms, altering constraints, or installing new algorithms. One expensive option is for the user to discard the existing instrument and replace it with a new instrument that includes the updated information. Another option, which is time-consuming and expensive, is to return the existing instrument to a manufacturing site that updates the new information to the processor. The manufacturing sites generally use expensive and complicated equipment including computers.

US 5,077,476 discloses an instrument for non-invasive measurement of blood glucose. A cartridge stores data unique to an individual patient/user. The cartridge is replaceable and is plugged into the analysis instrument through a receiving port. The cartridge includes electronic memory storage devices for storing data regarding an individual user and instrument software. The memory device is adapted to store a series of glucose readings, the time and the date of all measurements made by the analysis instrument, the total number of measurements made using a particular battery, as well as calibration constants for use by data processor. The memory device may be an EPROM-type chip that contains the software for operating the analytical instrument, thus facilitating re-programming of instrument by inserting an updated cartridge. In certain embodiments the cartridge also comprises a battery for supplying power to the near-infrared quantitative analysis instrument. The memory device and the battery are in electrical communication with the processing means via interface. US 5,077,476 fails to contemplate using an upload or reprogramming sequence to actually change the instrument and allow the instrument to operate according to a new program without requiring the memory device to be coupled to the instrument.

WO2000027276A1 discloses an in vivo imaging device having an illumination system that creates a virtual source within a tissue region of a subject in a non-invasive manner. The imaging device includes a computer system 1100 having one or more processors, such as processor 1104. Computer system 1100 also includes a main memory 1108, preferably random access memory (RAM), and can also include a secondary memory 1110. Secondary memory 1110 can include, for example, a hard disk drive 1112and/or a removable storage drive 1114, representing a floppy disk drive, a magnetic tape drive, an optical disk drive, etc. Removable storage drive 1114 reads from and/or writes to a removable storage unit 1118 in a well-known manner. Removable storage unit 1118 represents a floppy disk, magnetic tape, optical disk, etc. which is read by and written to by removable storage drive 1114. As will be appreciated, removable storage unit 1118 includes a computer usable storage medium having stored therein computer software and/or data. In alternative embodiments, secondary memory 1110 may include other similar means for allowing computer programs or other instructions to be loaded into computer system 1100. Such means can include, for example, a removable storage unit 1122 and an interface 1120. Examples of such can include a program cartridge and cartridge interface (such as that found in video game devices), a removable memory chip (such as an EPROM, or PROM) and associated socket, and other removable storage units 1122 and interfaces 1120 which allow software and data to be transferred from removable storage unit 1122 to computer system 1100. WO2000027276A1 is completely silent on receiving the information type and the updated information from a memory-update device, identifying the information type, and initiating an upload or reprogramming sequence according to the information type. In particular WO2000027276A1 does not disclose how the software type is identified and how the identification of the software type affects the manner in which the software type is loaded.

EP640978A2 discloses and apparatus making use of the existing data collection probe connector on the instrumentation as the port through which the software updates are loaded into the programmable memory devices that are used to store the operational software of the instrumentation. Circuitry is provided in the instrumentation to automatically differentiate between software update data being loaded into the instrumentation and the normal monitoring data that is received from the probe. This is accomplished by the use of probe defining circuitry that is able to differentiate between the standard probe used for data collection purposes and the software update probe that is provided to download software into the programmable memory devices. EP640978A2 fails to disclose receiving the information type and the updated information from the memory-update device, identifying the information type, and initiating an upload or reprogramming sequence according to the information type.

It would be desirable to overcome the above-noted problems, while still being able to provide updated information to the instrument with little or no interaction by the user.

### SUMMARY OF THE INVENTION

According to the invention an instrument for determining a concentration of an analyte in a sample, comprises a processor for determining a measurement of a concentration of an analyte in a sample; a communication device coupling the processor to a memory-update device; the memory-update device storing, on a memory, updated information to be installed on the instrument, the memory-update device stores, on the memory, an information type associated with the updated information, and the processor receives the information type and the updated information from the memory-update device and identifies the information type associated with the updated information, and initiates an upload or reprogramming sequence for the updated information according to the information type, the upload or reprogramming sequence allowing the processor to operate according to the updated information after the memory-update device is decoupled from the processor.

### Brief Description of the Drawings

FIG. 1a is a top view of a programming strip according to one embodiment of the present invention.
FIG. 1b is a side view of FIG. 1a.
FIG. 2 is a perspective view of a sensor-dispensing instrument in the open position showing a sensor pack being inserted according to one embodiment.
FIG. 3a is a front view of a sensor-dispensing instrument according to one embodiment.
FIG. 3b is a front view of a disposable cartridge with a programming strip and a plurality of reagent-test sensors according to one embodiment.
FIG. 3c is a top view of a reagent-test sensor according to one embodiment.
FIG. 4 is a front view of a sensor-dispensing instrument according to another embodiment.
FIG. 5a is a top view of a programming strip being inserted into an opening of the sensor-dispensing instrument of FIG. 4.
FIG. 5b is a side view of a programming strip being inserted into an opening of the sensor-dispensing instrument of FIG. 4.

While the invention is susceptible to various modifications and alternative forms, specific embodiments are shown by way of example in the drawings and are described in detail herein. It should be understood, however, that the invention is not intended to be limited to the particular forms disclosed. Rather, the invention is to cover all modifications, equivalents, and alternatives falling within the scope of the invention as defined by the appended claims.

### DETAILED DESCRIPTION OF THE ILLUSTRATED EMBODIMENTS

A programming strip is adapted to provide updated information to a processor (*e.g.,* a microprocessor) of an instrument. The programming strip may include updated information related to reprogramming the existing algorithms, altering constants, or installing new algorithms in the instrument. Some non-limiting examples of information that may be sent to the processor include: (a) amending or replacing at least one of the existing algorithms that determines the concentration of the analyte; (b) adding or amending software code to address bugs in the software; (c) altering constants in the existing algorithms; and (d) altering limits in the programming such as amending the minimum amount of fluid needed to determine the analyte concentration. The instruments may be of various types including sensor-dispensing instruments. The instruments may be portable or table-top instruments.

The instruments are typically used to determine concentrations of analytes. Analytes that may be measured by the instrument include glucose, lipid profiles (*e.g.,* cholesterol, triglycerides, LDL and HDL), microalbumin, hemoglobin A₁C, fructose, lactate, or bilirubin. The instruments are not limited, however, to determining these specific analytes and it is contemplated that other analyte concentrations may be determined. The analytes may be in, for example, a whole blood sample, a blood serum sample, a blood plasma sample, or other body fluids like ISF (interstitial fluid) and urine.

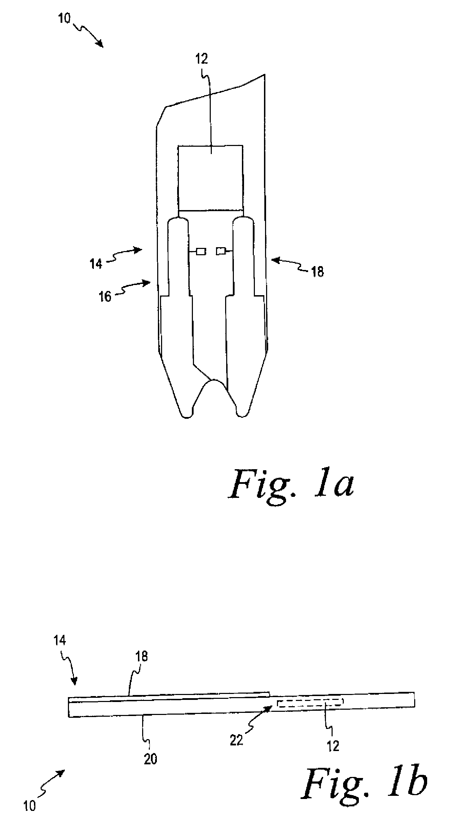

Referring to FIGs. 1a, b, a programming strip 10 is shown according to one embodiment. The programming strip 10 comprises a non-volatile memory 12 and a communication bus 14. In the embodiment illustrated, the communication bus 14 of FIG. 1 includes a power-data communication line 16 and a ground line 18. The non-volatile memory 12 stores the updated information and is communicatively coupled with a processor instrument via the communication bus 14.

A non-limiting example of non-volatile memory is an EEPROM. EEPROM (electronically erasable programmable read only memory) is a rewritable memory that does not require power to retain the contents of its memory. One example of memory that may be used in the programming strip is a chip marketed under the tradename DS2431, which is a 1024-bit, 1-wire® EEPROM chip made by Maxim Integrated Products, Inc./Dallas Semiconductor Corporation. Maxim Integrated Products is based in Sunnyvale, California, and Dallas Semiconductor Corporation is based in Dallas, Texas. The 1-wire® EEPROM chip is desirable because it uses only two wires. It is contemplated that other 1-wire® EEPROM chips may be used as the non-volatile memory.

It is contemplated that other EEPROMs may be used in the programming strip. It is contemplated that other non-volatile memory may be used in the programming strip, such as flash memory.

As shown in FIG. 1, the communication bus 14 is adapted to communicatively couple the memory 12 and the processor of the instrument. More specifically, according to one embodiment, the communication bus 14 is adapted to couple the memory 12 to the processor via a plurality of electrical connection pads of the instrument. The power-data communication line 16 enables two-way communication between the memory 12 and the processor of the instrument.

The communication bus 14 may be formed from a variety of materials. According to one embodiment, the communication bus comprises a plurality of traces. The plurality of traces may be an applied coating or painting such as carbon film. Alternative, the plurality of traces may be an applied coating or painting made of metal such as copper, tin, silver, gold, or combinations thereof. It is contemplated that the copper, tin, silver and gold may be in the form of alloys with other material. According to another embodiment, the communication bus may be a plurality of wires.

The communication bus 14 may have exactly two lines such as shown in FIG. 1a. It is contemplated that additional lines may be used to communicatively couple the memory and the processor of the instrument. For example, it is contemplated that exactly five lines may be used to communicatively couple the memory and the processor of the instrument.

The programming strip 10 typically is made of a polymeric material in which the memory and the conductive elements are placed thereon or therein. For example, the programming strip may be configured such as shown in FIG. 1b. As shown in FIG. 1b, the communication bus 14 is shown as being formed on top of the polymeric blank 20 and the non-volatile memory 12 is shown as being placed or embedded into an indentation 22. To further protect the non-volatile memory 12, it is desirable for the non-volatile memory to be placed into and mounted within the indentation 22. The non-volatile memory may be mounted in the indentation using, for example, an adhesive (*e.g.,* an epoxy) or a soldering process. It is contemplated, however, that the non-volatile memory may reside on a top surface of the polymeric blank. In this embodiment, the non-volatile memory may be attached using, for example, an adhesive (*e.g.,* an epoxy) or a soldering process. It is contemplated that the communication bus and memory may be formed or placed on the blank by other techniques.

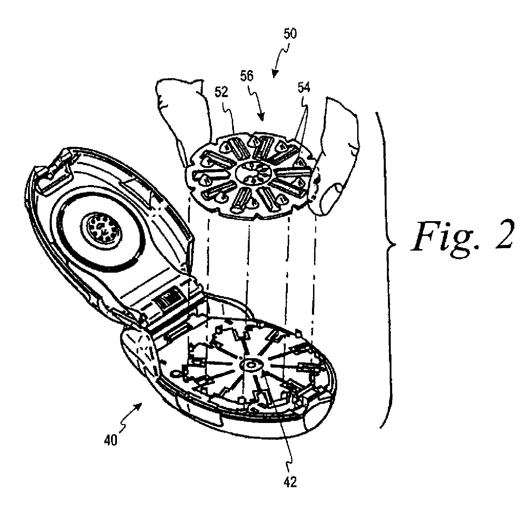

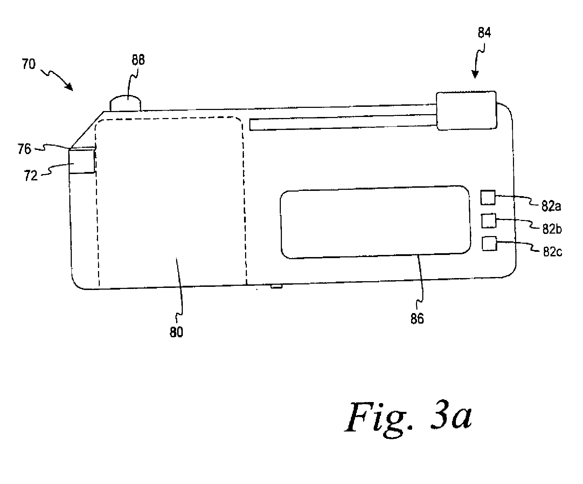

The memory 12 of the programming strip 10 is adapted to store the updated information and is adapted to be communicatively coupled with the instrument. According to one embodiment, the instrument is a sensor-dispensing instrument. Examples of sensor-dispensing instruments 40, 70 are shown in FIGs. 2,3a. It is contemplated that other sensor-dispensing instruments may be employed other than those depicted in FIGs. 2,3a.

More specifically, the memory 12 of the programming strip 10 is adapted to store the updated information and is adapted to be communicatively coupled with a processor (*e.g.,* a microprocessor) of the instrument. The processor performs the computing, which includes interpreting and executing the instructions. The processor also may be a central processing unit (CPU).

Referring to FIGs. 2, 3a, each of the sensor-dispensing instruments 40, 70 includes a respective processor 42, 72. One example of a processor that may be used is an uPD78F0338 microprocessor by NEC Corporation of Japan. It is contemplated that other processors may be used such as selected processors made by companies such as Texas Instruments, Inc., Intel Corporation and Siemens AG. It is also contemplated that other processors may be used in the instrument.

The processors 42, 72 are adapted to identify the programming strip 10 as an internal memory-update device. More specifically, the processors 42, 72 read the memory 12, which results in identifying the type of updated information to be installed and the proper upload or reprogramming sequence may be initiated. After the update has been completed, the processor of the instrument will have the latest information.

According to one embodiment, the programming strip may be individually stored. The programming strip may be stored in a container such as a bottle or vial. The programming strip may also be packaged in a bottle or vial with a plurality of test sensors. According to one embodiment, the bottle contains one programming strip and from about 5 to about 100 test sensors. It is contemplated that the programming strip may be individually stored in other containers such as a packet.

According to another embodiment, the programming strip may be located in a disposable cartridge such as a sensor pack (*e.g.,* a blister-type pack) by replacing one of the test sensors that is adapted to determine the analyte concentration. According to one embodiment, the test sensors are reagent-test sensors.

One example of a sensor pack or, more specifically, a blister-type pack is shown in FIG. 2. The blister-type pack 50 is shown in FIG. 2 being placed in the sensor-dispensing instrument 40. The blister-type pack 50 includes a programming strip 52 and a plurality of test sensors 54 that are individually stored in a respective one of the sensor cavities 56. It is contemplated that other sensor packs that individually hold the programming strip and plurality of test sensors may be used. The sensor pack of FIG. 2 (without the programming strip) is further described at U.S. Publication No. 2003/0032190 that published on February 13, 2003 and is entitled "Mechanical Mechanism for a Blood Glucose Sensor-Dispensing Instrument." It is contemplated that other sensor packs may be used.

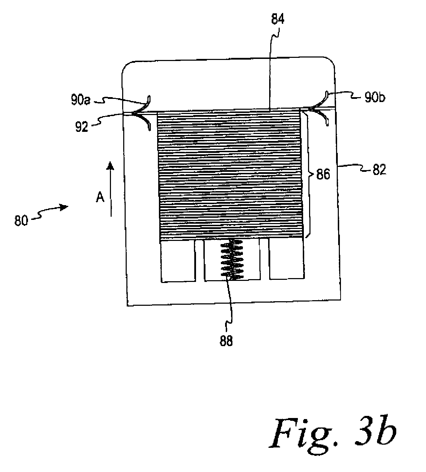

According to a further embodiment, the programming strip may also be located in a stack with a plurality of test sensors within a disposable cartridge such as shown in FIG. 3b. Referring to FIG. 3b, a disposable cartridge 80 includes a housing 82, a programming strip 84, and a plurality of reagent-test sensors 86. The programming strip 84 and the plurality of reagent-test sensors 86 is stacked in the cartridge 80.

The programming strip 84 and the plurality of stacked reagent-test sensors 86 are moved in the direction of arrow A via a spring 88. The cartridge 80 also includes a plurality of seals 90a,b that protects the stacked reagent-test sensors 90 from the humidity. The programming strip 84 and the plurality of reagent-test sensors 86, one at a time, exit the cartridge 80, via an opening 92. To promptly provide the new information to the processor of the instrument, the programming strip 84 is desirably located to be removed first from the cartridge 80.

The disposable cartridge 80 of FIG. 3b may be stored in the sensor-dispensing instrument 70 of FIG. 3a. It is contemplated that other cartridges may be used to contain the programming strip and the plurality of test sensors.

Typically, the sensor pack 50 and cartridge 80 of FIGs. 2,3b contain only one programming strip because all the updated information is stored in the memory of the programming strip. The cartridges typically contain from about 10 to about 50 test sensors and, more specifically, contain from about 25 to about 40 test sensors.

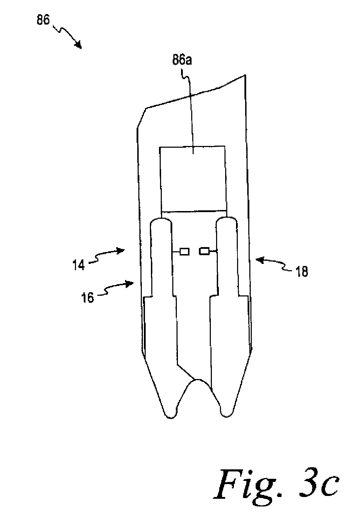

To reduce complexity, it may be desirable for the dimensions of the programming strip to be similar, if not identical, to the plurality of the test sensors that is adapted to determine the analyte concentration. For example, in one illustrated embodiment, the dimensions of the programming strip 10 of FIG. 1 are the same as the reagent-test sensors 86 shown in FIG. 3c. Specifically, the only difference between the programming strip 10 of FIG. 1 and the reagent-test sensors 86 of FIG. 3c is the replacement of the reagent-receiving area 86a with the memory 12 to form the programming strip 10. The reagent-receiving area and the memory may also have the same dimensions. According to another embodiment, the programming strip and the reagent-test sensors may be dimensioned differently. Similarly, the dimensions of the reagent-receiving area and the memory be different.

According to the methods disclosed herein updated information may be provided to a processor of the instrument by a user. The user may be, for example, a user at home who needs to determine an analyte concentration (e.g., glucose) via the instrument.

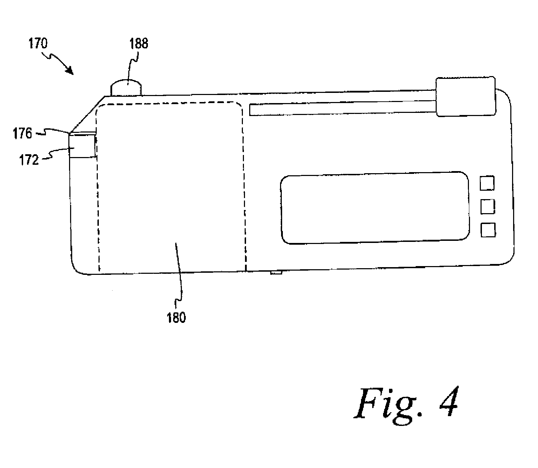

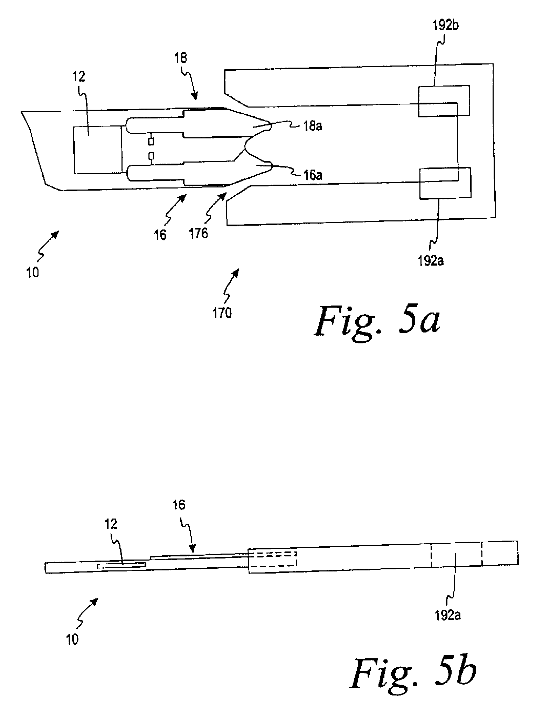

To provide the updated information to the processor of the instrument, the programming strip needs to be properly positioned in the instrument such that the programming strip is in communicatively coupled with the processor. According to one method, a user grasps the programming strip and properly positions it in an opening of the instrument. For example, referring to FIGs. 4 and 5a,b, a user may take a programming strip (*e.g.,* programming strip 10) and position it in an opening 176 of sensor-dispensing instrument 170. The memory 12 of the programming strip 10 would then be communicatively coupled with the processor 172 of the sensor-dispensing instrument 170. In the illustrated embodiment, end portions 16a, 18a would contact a plurality of electrical contact pads 192a,b of the instrument 170 when the programming strip 10 is properly positioned in the opening 176. In the embodiment of FIG. 4, a cartridge 180 would not likely include a programming strip. Rather, the cartridge 180 includes only a plurality of test sensors.

To reduce costs, it is desirable to use an opening adapted to receive both the programming strip and the plurality of test sensors for determining the analyte concentration such as shown in FIG. 4 with opening 176. Additionally, by using the opening for both the programming strip and the test sensors, the instrument may be more compact by avoiding the need to have an opening for only the programming strip. It is contemplated, however, that separate openings adapted to receive a respective programming strip and test sensors may be formed in the instrument.

According to another method, the programming strip may automatically be moved and properly positioned without the user handling the programming strip. For example, referring to FIGs. 3a,3b, after a user positions the cartridge 80 in the sensor-dispensing instrument, the sensor-dispensing instrument automatically advances the programming strip 84 into an opening 76. The automatic advancing of the programming strip 84 may be initiated by several methods. For example, the automatic advancing may be initiated by pressing one of the plurality of buttons 82a-c, or pushing a pusher mechanism 84. The programming strip may be advanced by using a motor. It also may be initiated by the placing of the cartridge within the sensor-dispensing instrument.

The process of providing information from the memory of the programming strip to the processor of the instrument is typically performed in a short time period. An example of a short time period is generally from about 0.1 millisecond ("ms") to about 1 second and, more typically, from about 1 ms to about 50 ms. It is contemplated that the time needed to forward the updated information from the memory of the programming strip to the processor of the instrument may take longer, but this is, of course, less desirable.

According to one embodiment, the sensor-dispensing instrument 40, 70 may notify the user that an update or reprogramming sequence has occurred and the programming strip (*e.g.,* programming strip 10) may be removed or discarded. For example, a display 86 of the sensor-dispensing instrument 70 in FIG. 3a may notify the user that an update sequence has occurred. One example of a display is a liquid-crystal display.

It is contemplated that the programming strip may be removed automatically by the instrument via an eject mechanism. In such a method, the test sensor is released forcefully. According to another method, a user manually releases the programming strip via a release mechanism 88 (FIG. 3a) or a release mechanism 188 (FIG. 4). In such embodiments, after the release mechanism is activated, the test sensors may be removed by tipping the instrument 70, 170 such that the programming strip falls from the instrument 70, 170 via gravity. Alternatively, after the release mechanism is activated, the test sensor may be removed by pulling it from the instrument. It is contemplated that the test sensor may be pulled from the instrument without using a release mechanism. It is contemplated that the programming strip may be removed by other techniques. After completing the reprogramming, the sensor-dispensing instrument will function as intended with, for example, the updated program, constants or algorithms.

The programming strip of embodiment A wherein the communication bus comprises exactly five lines.

### Embodiment D

The programming strip of embodiment A wherein the non-volatile memory is an EEPROM.

### Embodiment E

The programming strip of embodiment A wherein the non-volatile memory is a flash memory.

### Embodiment F

The programming strip of embodiment A wherein the programming strip forms an indentation that receives the non-volatile memory.

### Embodiment G

A programming strip that is adapted to provide updated information to a processor of an instrument, the instrument being adapted to determine the concentration of an analyte, the programming strip comprising:
non-volatile memory being adapted to store the updated information and being adapted to be communicatively coupled with the processor of the instrument, the non-volatile memory being an EEPROM; and
a communication bus having exactly two lines that are adapted to assist in communicatively coupling the memory and the processor of the instrument, the first line being a power-data communication line and the second line being a ground line.

### Embodiment H

The programming strip of embodiment G wherein the communication bus comprises a plurality of traces.

### Embodiment I

The programming strip of embodiment G wherein the programming strip forms an indentation that receives the EEPROM.

### Embodiment J

A programming strip that is adapted to provide updated information to a processor of an instrument, the instrument being adapted to determine the concentration of an analyte, the programming strip comprising:
non-volatile memory being adapted to store the updated information and being adapted to be communicatively coupled with the processor of the instrument, the non-volatile memory being a flash memory; and
a communication bus having exactly two lines that are adapted to assist in communicatively coupling the memory and the processor of the instrument, the first line being a power-data communication line and the second line being a ground line.

### Embodiment K

The programming strip of embodiment J wherein the communication bus comprises a plurality of traces.

### Embodiment L

The programming strip of embodiment J wherein the programming strip forms an indentation that receives the flash memory.

### Embodiment M

A cartridge that is adapted to be used in a sensor-dispensing instrument, the instrument being adapted to determine the concentration of an analyte and includes a processor, the cartridge comprising a programming strip and a plurality of test sensors, the programming strip including non-volatile memory and a communication bus, the non-volatile memory being adapted to store updated information and being adapted to be communicatively coupled with the processor of the instrument, the communication bus having at least two lines that are adapted to assist in communicatively coupling the memory and the processor of the instrument.

### Embodiment N

The cartridge of embodiment M wherein the plurality of test sensors is reagent-test sensors.

### Embodiment O

The cartridge of embodiment M wherein the cartridge is a sensor pack.

### Embodiment P

The cartridge of embodiment O wherein the sensor pack is a blister-type pack.

### Embodiment Q

The cartridge of embodiment M wherein the plurality of test sensors is stacked.

### Embodiment R

The cartridge of embodiment Q further including at least one seal to assist in protecting the plurality of test sensors.

### Embodiment S

The cartridge of embodiment M wherein the programming strip and the plurality of test sensors have the same dimensions.

### Embodiment T

A cartridge that is adapted to be used in a sensor-dispensing instrument, the instrument being adapted to determine the concentration of an analyte and includes a processor, the cartridge comprising a programming strip and a plurality of test sensors, the programming strip including non-volatile memory and a communication bus, the non-volatile memory being adapted to store updated information and being adapted to be communicatively coupled with the processor of the instrument, the communication bus having exactly two lines that are adapted to assist in communicatively coupling the memory and the processor of the instrument, the first line being a power-data communication line and the second line being a ground line.

### Embodiment U

The cartridge of embodiment T wherein the non-volatile memory is an EEPROM.

### Embodiment V

The cartridge of embodiment T wherein the non-volatile memory is a flash memory.

### Embodiment W

The cartridge of embodiment T wherein the plurality of test sensors is reagent-test sensors.

### Embodiment X

The cartridge of embodiment T wherein the cartridge is a sensor pack.

### Embodiment Y

The cartridge of embodiment X wherein the sensor pack is a blister-type pack.

### Embodiment Z

The cartridge of embodiment T wherein the plurality of test sensors is stacked.

### Embodiment AA

The cartridge of embodiment Z further including at least one seal to assist in protecting the plurality of test sensors.

### Embodiment BB

The cartridge of embodiment T wherein the programming strip and the plurality of test sensors have the same dimensions.

### Embodiment CC

An instrument for determining the analyte concentration of a fluid, the instrument comprising:
a programming strip including non-volatile memory and a communication bus, the non-volatile memory being adapted to store updated information, the communication bus having at least two lines;
an opening adapted to receive at least the programming strip; and
a processor adapted to receive information from the non-volatile memory of the programming strip after the programming strip has been positioned at least partially in the opening such that the programming strip and processor are communicatively coupled via the communication bus.

### Embodiment DD

The instrument of embodiment CC further including a plurality of test sensors.

### Embodiment EE

The instrument of embodiment DD wherein the opening is adapted to receive both the programming strip and the plurality of test sensors.

### Embodiment FF

The instrument of embodiment DD wherein the plurality of test sensors is reagent-test sensors.

### Embodiment GG

The instrument of embodiment CC wherein the fluid is blood and the analyte is glucose.

### Embodiment HH

The instrument of embodiment CC further including a plurality of electrical connection pads that is adapted to assist in communicatively coupling the programming strip and the processor.

### Embodiment II

An instrument for determining the analyte concentration of a fluid, the instrument comprising:
a programming strip including non-volatile memory and a communication bus, the non-volatile memory being adapted to store the updated information, the communication bus having exactly two lines, the first line being a power-data communication line and the second line being a ground line;
an opening adapted to receive at least the programming strip; and
a processor adapted to receive information from the non-volatile memory of the programming strip after the programming strip has been positioned at least partially in the opening such that the programming strip and processor are communicatively coupled via the communication bus.

### Embodiment JJ

The instrument of embodiment II wherein the non-volatile memory is an EEPROM.

### Embodiment KK

The instrument of embodiment II wherein the non-volatile memory is a flash memory.

### Embodiment LL

The instrument of embodiment II further including a plurality of test sensors.

### Embodiment MM

The instrument of embodiment LL wherein the opening is adapted to receive both the programming strip and the plurality of test sensors.

### Embodiment NN

The instrument of embodiment LL wherein the plurality of test sensors is reagent-test sensors.

### Embodiment OO

The instrument of embodiment II wherein the fluid is blood and the analyte is glucose.

### Embodiment PP

The instrument of embodiment II further including a plurality of electrical connection pads that is adapted to assist in communicatively coupling the programming strip and the processor.

### Embodiment QQ

A method of updating information to an instrument adapted to determine the analyte concentration of a fluid, the method comprising the acts of:
providing the instrument including a programming strip, an opening adapted to receive at least the programming strip, and a processor, the programming strip including non-volatile memory and a communication bus, the non-volatile memory being adapted to store the updated information, the communication bus having at least two lines;
positioning the programming strip at least partially in the opening such that the programming strip and processor are communicatively coupled via the communication bus; and
updating the processor with the information stored in the non-volatile memory.

### Embodiment RR

The method of embodiment QQ wherein the positioning of the programming strip including a user grasping the programming strip and placing the programming strip at least partially in the opening.

### Embodiment SS

The method of embodiment QQ wherein the opening is adapted to receive the programming strip and the plurality of test sensors.

### Embodiment TT

The method of embodiment QQ wherein the positioning of the programming strip is performed automatically by the instrument without the user handling the programming strip.

### Embodiment UU

The method of embodiment QQ further including removing the programming strip from the opening.

### Embodiment VV

A method of updating information to an instrument adapted to determine the analyte concentration of a fluid, the method comprising the acts of:
providing the instrument including a programming strip, an opening adapted to receive at least the programming strip, and a processor, the programming strip including non-volatile memory and a communication bus, the non-volatile memory being adapted to store the updated information, the communication bus having exactly two lines, the first line being a power-data communication line and the second line being a ground line;
positioning the programming strip at least partially in the opening such that the programming strip and processor are communicatively coupled via the communication bus; and
updating the processor with the information stored in the non-volatile memory.

### Embodiment WW

The method of embodiment VV wherein the positioning of the programming strip including a user grasping the programming strip and placing the programming strip at least partially in the opening.

### Embodiment XX

The method of embodiment VV wherein the opening is adapted to receive the programming strip and the plurality of test sensors.

### Embodiment YY

The method of embodiment VV wherein the positioning of the programming strip is performed automatically by the instrument without the user handling the programming strip.

### Embodiment ZZ

The method of embodiment VV further including removing the programming strip from the opening.

### Embodiment AAA

The cartridge of embodiment VV wherein the non-volatile memory is an EEPROM.

### Embodiment BBB

The cartridge of embodiment VV wherein the non-volatile memory is a flash memory.

## Claims

1. An instrument (40, 70, 170) for determining a concentration of an analyte in a sample, the instrument comprising:
a processor (42, 72, 172) for determining a measurement of a concentration of an analyte in a sample; and
a communication device (14) coupling the processor to a memory-update device (10, 84), the memory-update device (10, 84) storing, on a memory (12), updated information to be installed on the instrument,
**characterized in that**
the updated information includes at least one of reprogramming information, altering information, or new installation information, and the memory-update device (10, 84) also stores, on the memory (12), an information type identifying which type of the updated information is stored on the memory, and the processor (42, 72, 172) receives the information type and the updated information from the memory-update device (10, 84), identifies the information type associated with the updated information stored on the memory, and initiates an upload sequence or a reprogramming sequence for the updated information according to the information type, the upload sequence or the reprogramming sequence allowing the processor (42, 72,172) to operate according to the updated information after completing the sequence, wherein the memory-update device (10, 84) is selectively decoupled from the processor (42, 72,172).

2. The instrument (40, 70, 170) of claim 1, wherein the processor (42, 72, 172) determines the concentration measurement at least according to the updated information.

3. The instrument (40, 70,170) of claim 1, wherein the processor (42, 72, 172) determines the concentration measurement according to at least one algorithm, and the upload or reprogramming sequence amends or replaces the at least one algorithm with the updated information.

4. The instrument (40, 70, 170) of claim 1, wherein the processor (42, 72, 172) determines the concentration measurement according to at least one algorithm, and the upload or reprogramming sequence alters at least one constant in the at least one algorithm.

5. The instrument (40, 70, 170) of claim 1, wherein the processor (42, 72, 172) executes software to determine the concentration measurement, and the upload or reprogramming sequence adds or amends software code that addresses bugs in the software.

6. The instrument (40, 70, 170) of claim 1, wherein the processor (42, 72, 172) determines the concentration measurement according to a program for the instrument (40, 70, 170), and the upload or reprogramming sequence alters at least one limit for the program.

7. The instrument (40, 70, 170) of claim 6, wherein the upload or reprogramming sequence alters a minimum amount of sample needed to determine the concentration measurement.

8. The instrument (40, 70, 170) of claim 1, further comprising a display (86) that provides a user with notification regarding the updated information to be installed on the instrument (40, 70, 170).

9. The instrument (40, 70, 170) of claim 8, wherein the notification indicates to the user actions to take regarding the memory-update device (10, 84).

10. The instrument (40, 70, 170) of claim 1, wherein the processor (42, 72, 172) reads the memory-update device (10, 84), identifies a type associated with the updated information, and initiates an upload or reprogramming sequence in a time period ranging from about 0.1 millisecond to about 1 second.

11. The instrument (40, 70, 170) of claim 1, wherein the memory (12) of the memory-update device (10, 84) includes a non-volatile memory.

12. The instrument (40, 70, 170) of claim 11, wherein the communication device (14) includes a power-data communication line (16) providing two-way communication between the processor (42, 72, 172) and the non-volatile memory (12).

13. The instrument (40, 70, 170) of claim 11, wherein the non-volatile memory (12) is included on a programming strip (10) that is received by the instrument (40, 70,170) and includes a communication bus (14) that connects the non-volatile memory (12) with the communication device (14) and the processor (42, 72, 172) on the instrument (40, 70, 170).

14. The instrument (40, 70, 170) of claim 1, wherein the communication device (14) includes electrical connection pads (192a, b) connecting the processor (42, 72, 172) and the memory-update device (10, 84).

15. The instrument (40, 70, 170) of claim 1, further comprising an opening (76,176) for receiving the memory-update device (10,84), wherein the opening (76, 176) further receives test sensors (86) that collect the sample.

## Patentansprüche

1. Instrument (40, 70, 170) zum Bestimmen einer Konzentration eines Analyten in einer Probe, wobei das Instrument umfasst:
einen Prozessor (42, 72, 172) zum Bestimmen einer Messung einer Konzentration eines Analyten in einer Probe; und
eine Kommunikationsvorrichtung (14), die den Prozessor mit einer Speicheraktualisierungsvorrichtung (10, 84) verbindet, wobei die Speicheraktualisierungsvorrichtung (10, 84) in einem Speicher (12) aktualisierte Informationen speichert, die in dem Instrument installiert werden sollen,
**dadurch gekennzeichnet, dass**
die aktualisierten Informationen mindestens ein Element aus Umprogrammierinformation, Änderungsinformation oder neuer Installationsinformation umfassen, und die Speicheraktualisierungsvorrichtung (10, 84) im Speicher (12) auch einen Informationstyp speichert, der identifiziert, welche Art von aktualisierten Information im Speicher gespeichert ist, und der Prozessor (42, 72, 172) die Informationsart und die aktualisierte Information von der Speicheraktualisierungsvorrichtung (10, 84) empfängt, die Informationsart identifiziert, die mit den aktualisierten Information assoziiert ist, welche im Speicher gespeichert ist, und eine Hochladesequenz oder eine Umprogrammierungssequenz für die aktualisierte Information gemäß der Informationsart initiiert, wobei die Hochladesequenz oder die Umprogrammierungssequenz dem Prozessor (42, 72, 172) ermöglichen, gemäß der aktualisierten Information nach dem Abschluss der Sequenz zu arbeiten, wobei die Speicheraktualisierungsvorrichtung (10, 84) selektiv vom Prozessor (42, 72, 172) entkoppelt ist.

2. Instrument (40, 70, 170) nach Anspruch 1, wobei der Prozessor (42, 72, 172) die Konzentrationsmessung zu mindestens gemäß der aktualisierten Information bestimmt.

3. Instrument (40, 70, 170) nach Anspruch 1, wobei der Prozessor (42, 72, 172) die Konzentrationsmessung gemäß mindestens einem Algorithmus bestimmt, und die Hochlade- oder Umprogrammierungssequenz den mindestens einen Algorithmus durch die aktualisierte Information modifiziert oder ersetzt.

4. Instrument (40, 70, 170) nach Anspruch 1, wobei der Prozessor (42, 72, 172) die Konzentrationsmessung gemäß mindestens einem Algorithmus bestimmt, und die Hochlade- oder Umprogrammierungssequenz mindestens eine Konstante in dem mindestens einen Algorithmus ändert.

5. Instrument (40, 70, 170) nach Anspruch 1, wobei der Prozessor (42, 72, 172) Software ausführt, um die Konzentrationsmessung zu bestimmen, und die Hochlade- oder Umprogrammierungssequenz Softwarecode hinzufügt oder denselben modifiziert, der Fehler in der Software betrifft.

6. Instrument (40, 70, 170) nach Anspruch 1, wobei der Prozessor (42, 72, 172) die Konzentrationsmessung gemäß einem Programm für das Instrument (40, 70, 170) bestimmt, und die Hochlade- oder Umprogrammierungssequenz mindestens einen Grenzwert für das Programm ändert.

7. Instrument (40, 70, 170) nach Anspruch 6, wobei die Hochlade- oder Umprogrammierungssequenz einen Mindestbetrag der Probe ändert, die benötigt wird, um die Konzentrationsmessung zu bestimmen.

8. Instrument (40, 70, 170) nach Anspruch 1, das ferner ein Display (86) umfasst, das einem Nutzer eine Mitteilung bezüglich der aktualisierten Information bereitgestellt, die im Instrument (40, 70, 170) installiert werden sollen.

9. Instrument (40, 70, 170) nach Anspruch 8, wobei die Mitteilung dem Nutzer Aktionen anzeigt, die bezüglich der Speicheraktualisierungsvorrichtung (10, 84) zu ergreifen sind.

10. Instrument (40, 70, 170) nach Anspruch 1, wobei der Prozessor (42, 72, 172) die Speicheraktualisierungsvorrichtung (10, 84) liest, eine Art identifiziert, die mit der aktualisierten Information verbunden ist, und eine Hochlade- oder Umprogrammierungssequenz in einer Zeitspanne initiiert, die von etwa 0,1 ms bis etwa 1 Sekunde reicht.

11. Instrument (40, 70, 170) nach Anspruch 1, wobei der Speicher (12) der Speicheraktualisierungsvorrichtung (10, 84) einen nichtflüchtigen Speicher umfasst.

12. Instrument (40, 70, 170) nach Anspruch 11, wobei die Kommunikationsvorrichtung (14) eine Leistungsdaten-Kommunikationsleitung (16) umfasst, die eine Zwei-Wege-Kommunikation zwischen dem Prozessor (42, 72, 172) und dem nichtflüchtigen Speicher (12) bereitstellt.

13. Instrument (40, 70, 170) nach Anspruch 11, wobei der nichtflüchtige Speicher (12) von einem Programmierungsabschnitt (10) umfasst ist, der von dem Instrument (40, 70, 170) empfangen wird und einen Kommunikationsbus (14) umfasst, der den nichtflüchtigen Speicher (12) mit der Kommunikationsvorrichtung (14) und dem Prozessor (42, 72, 172) in dem Instrument (40, 70, 170) verbindet.

14. Instrument (40, 70, 170) nach Anspruch 1, wobei die Kommunikationsvorrichtung (14) elektrische Verbindungspads (192a, b) umfasst, die den Prozessor (42, 72, 172) und die Speicheraktualisierungsvorrichtung (10, 84) verbinden.

15. Instrument (40, 70, 170) nach Anspruch 1, das ferner eine Öffnung (76, 176) zum Aufnehmen der Speicheraktualisierungsvorrichtung (10, 84) umfasst, wobei die Öffnung (76, 176) ferner Testsensoren (86) aufnimmt, die die Probe sammeln.

## Revendications

1. Instrument (40, 70, 170) pour déterminer une concentration d'un analyte dans un échantillon, l'instrument comprenant :
un processeur (42, 72, 172) pour déterminer une mesure d'une concentration d'un analyte dans un échantillon ; et
un dispositif de communication (14) couplant le processeur à un dispositif de mise à jour de la mémoire (10, 84), le dispositif de mise à jour de la mémoire (10, 84) stockant, sur une mémoire (12), des informations mises à jour devant être installées sur l'instrument,
**caractérisé en ce que**
les informations mises à jour comprennent au moins l'une de celles parmi des informations de reprogrammation, des informations de modification ou de nouvelles informations d'installation, et le dispositif de mise à jour de la mémoire (10, 84) stocke également, sur la mémoire (12), un type d'informations identifiant quel type des informations mises à jour est stocké sur la mémoire, et
le processeur (42, 72, 172) reçoit du dispositif de mise à jour de la mémoire (10, 84), le type d'informations et les informations mises à jour, identifie le type d'informations associé aux informations mises à jour et stockées sur la mémoire, et initie une séquence de téléchargement ou une séquence de reprogrammation prévue pour les informations mises à jour conformément au type d'informations, la séquence de téléchargement ou la séquence de reprogrammation permettant au processeur (42, 72, 172) de fonctionner conformément aux informations mises à jour, après achèvement de la séquence au cours de laquelle le dispositif de mise à jour de la mémoire (10, 84) est sélectivement découplé du processeur (42, 72, 172).

2. Instrument (40, 70, 170) selon la revendication 1, dans lequel le processeur (42, 72, 172) détermine la mesure de la concentration au moins conformément aux informations mises à jour.

3. Instrument (40, 70, 170) selon la revendication 1, dans lequel le processeur (42, 72, 172) détermine la mesure de la concentration conformément à au moins un algorithme, et la séquence de téléchargement ou de reprogrammation modifie ou remplace, avec ou par les informations mises à jour, l'algorithme au moins au nombre de un.

4. Instrument (40, 70, 170) selon la revendication 1, dans lequel le processeur (42, 72, 172) détermine la mesure de la concentration conformément à au moins un algorithme, et la séquence de téléchargement ou de reprogrammation modifie au moins une constante dans l'algorithme au moins au nombre de un.

5. Instrument (40, 70, 170) selon la revendication 1, dans lequel le processeur (42, 72, 172) exécute un logiciel pour déterminer la mesure de la concentration, et la séquence de téléchargement ou de reprogrammation ajoute ou modifie un code de logiciel qui accède à des erreurs contenues dans le logiciel.

6. Instrument (40, 70, 170) selon la revendication 1, dans lequel le processeur (42, 72, 172) détermine la mesure de la concentration conformément à un programme prévu pour l'instrument (40, 70, 170), et la séquence de téléchargement ou de reprogrammation modifie au moins une limite pour le programme.

7. Instrument (40, 70, 170) selon la revendication 6, dans lequel la séquence de téléchargement ou de reprogrammation modifie une quantité minimum de l'échantillon nécessaire pour déterminer la mesure de la concentration.

8. Instrument (40, 70, 170) selon la revendication 1, comprenant en outre un affichage (86) qui fournit à un utilisateur une notification concernant les informations mises à jour devant être installées sur l'instrument (40, 70, 170).

9. Instrument (40, 70, 170) selon la revendication 8, dans lequel la notification indique à l'utilisateur les actions à entreprendre concernant le dispositif de mise à jour de la mémoire (10, 84).

10. Instrument (40, 70, 170) selon la revendication 1, dans lequel le processeur (42, 72, 172) lit le dispositif de mise à jour de la mémoire (10, 84), identifie un type associé aux informations mises à jour et initie une séquence de téléchargement ou de reprogrammation dans une période de temps comprise entre 0,1 milliseconde environ et 1 seconde environ.

11. Instrument (40, 70, 170) selon la revendication 1, dans lequel la mémoire (12) du dispositif de mise à jour de la mémoire (10, 84) comprend une mémoire non volatile.

12. Instrument (40, 70, 170) selon la revendication 11, dans lequel le dispositif de communication (14) comprend une ligne de communication de données de puissance (16) fournissant une communication dans les deux sens, entre le processeur (42, 72, 172) et la mémoire non volatile (12).

13. Instrument (40, 70, 170) selon la revendication 11, dans lequel la mémoire non volatile (12) est contenue sur une bande de programmation (10) qui est reçue par l'instrument (40, 70, 170) et qui comprend un bus de communication (14) qui connecte la mémoire non volatile (12) au dispositif de communication (14) et au processeur (42, 72, 172), sur l'instrument (40, 70, 170).

14. Instrument (40, 70, 170) selon la revendication 1, dans lequel le dispositif de communication (14) comprend des touches de connexion électrique (192a, b) connectant le processeur (42, 72, 172) et le dispositif de mise à jour de la mémoire (10, 84).

15. Instrument (40, 70, 170) selon la revendication 1, comprenant en outre une ouverture (76, 176) pour recevoir le dispositif de mise à jour de la mémoire (10, 84), instrument dans lequel l'ouverture (76, 176) reçoit en outre des capteurs de tests (86) qui collectent l'échantillon.
